# EUROPEAN PATENT APPLICATION

(11) **EP 4 349 368 A1**
(43) Date of publication of application: **10.04.2024**
(21) Application number: 22815998.4
(22) Date of filing: 27.05.2022
(51) Int. Cl.: A61K 39/395, A61K 45/00, A61P 35/00, A61P 43/00, C07K 16/18, C07K 16/28

(54) **THERAPY USING COMBINATION OF ANTI-CD26 ANTIBODY AND IMMUNE CHECKPOINT INHIBITOR**

(30) Priority: 31.05.2021 JP 2021091761
(71) Applicant: Y'S AC Co., Ltd., Tokyo 150-0021 (JP); Juntendo Educational Foundation, Tokyo 113-8421 (JP)
(72) Inventor: MORIMOTO, Chikao, Tokyo 156-0043 (JP); HATANO, Ryo, Yokohama-shi, Kanagawa 220-0042 (JP); OHNUMA, Kei, Tokyo 141-0032 (JP); KANEKO, Yutaro, Tokyo 150-0021 (JP)
(74) Representative: Dennemeyer & Associates S.A.
(86) International application number: PCT/JP2022/021738
(87) International publication number: WO 2022/255248

(57) **Abstract**

An object of the present disclosure is to develop a new combined therapy that exhibits an excellent antitumor effect as compared with administration of an anti-CD26 antibody alone. In order to evaluate both a function of an immune checkpoint inhibitor and an antitumor effect of the anti-CD26 antibody, it is necessary to include both an immune system syngeneic with a cancer cell and a binding site on a human CD26 molecule. That is, in order to acquire data of the immune checkpoint inhibitor and the anti-CD26 antibody, analysis in a human tumor cell line and a human immune system is essential. Therefore, the present inventors tried to produce a human immunized mouse as a model animal for confirming a combined action of the anti-CD26 antibody and the ICI, and succeeded in producing a model mouse excellent in generation of human T cells. Subsequently, in a cancer-bearing model using this mouse, a combined effect of the anti-CD26 antibody and the immune checkpoint inhibitor was studied. As a result, it has been found that this combined drug has a stronger synergistic effect than each single drug.

## Description

### Cross-Reference to Related Applications

The present international application claims priority based on Japanese Patent Application No. 2021-091761 filed with the Japanese Patent Office on May 31, 2021, and the entire contents of Japanese Patent Application No. 2021-091761 are incorporated herein by reference.

### Technical Field

The present disclosure relates to a field of treatment of a cancer patient.

### Background Art

Malignant pleural mesothelioma is a refractory malignant tumor derived from pleural mesothelioma caused by exposure to asbestos. A latent period from exposure to asbestos to onset is said to be about 30 years, and it is thought that the number of patients will increase more and more in the future in Asia and the Middle East including Japan, Chin, and India. Prognosis is extremely poor, and a surgery therapy, a chemotherapy, a radiation therapy, and the like are performed, but all of these do not provide satisfactory therapy results, and establishment of a new therapy is desired. In addition, a lung cancer is a cancer type with the largest number of patients in Japan, a 5-year survival ratio is as low as about 20%, and establishment of a new safe and effective therapy for the lung cancer is also required.

An anti-CD26 antibody is known to have an anticancer effect (Patent Literatures 1 and 2). A First-in-Human phase I clinical test was performed in France focusing on malignant mesothelioma. It is reported that there was no side effect to be noted except for Infusion reaction (acute infusion reaction), safety was confirmed, ten cases out of 19 cases of malignant mesothelioma patients resistant to an anticancer drug were determined as Stable Disease (SD) by modified RESIST evaluation, five cases of which continued SD for six months or more and up to 399 days, and a result suggesting efficacy was also obtained (Non Patent Literature 1). Also in Japan, a phase I/II clinical test on malignant mesothelioma resistant to an anticancer drug was performed. In phase I, administration was performed to a total of nine cases including three cases for each of cohort 1 to cohort 3. In phase II, administration was performed to 31 cases. Administration to a final patient in phase II was completed in 2019. Also in domestic clinical tests, partial response (PR)·SD is achieved at a high ratio with respect to malignant mesothelioma patients resistant to an anticancer drug, and an antitumor effect is recognized (Non Patent Literature 2). Meanwhile, there was no complete response (CR), and there was a patient who became Progressive Disease (PD) from SD in a relatively short period of time, and thus development of a therapy using the present antibody that exhibits a longer antitumor effect and gives a progression-free survival time is an important object.

Until now, attempts have been made to enhance a treatment effect by combined use of an anti-CD26 antibody and a small molecule compound (Patent Literature 3).

An immune checkpoint inhibitor (ICI) is a generic term for drugs that inhibit binding between an inhibitory receptor expressed in immune cells, particularly T cells, and a ligand thereof to inhibit transmission of an inhibitory signal to immune cells and subsequent functional suppression. Examples of representative ICIs already approved include an anti-cytotoxoc T-lymphocyte associated ptotein 4 (CTLA-4) antibody, an anti-Programmed cell death 1 (PD-1) antibody, and an anti-Programmed cell death-ligand 1 (PD-L1) antibody. The ICI is effective in various cancer types such as malignant melanoma, non-small cell lung cancer, renal cell cancer, rectal cancer, and Hodgkin's lymphoma. However, there is a report that an ICI prolongs an asymptomatic period by use thereof alone, but it is difficult to achieve complete response (CR). Therefore, attempts have been made to supplement the treatment effect of the ICI by combined use with another drug. For example, in order to enhance a treatment effect on non-small cell lung cancer, combined use of an anti-PD-1 antibody and an anti-CTLA-4 antibody has been attempted, but regarding the treatment effect, there are reports that there is an additive effect, and there are reports that no additive effect is recognized, and the results are various. Meanwhile, it is a problem that toxicity due to combined use is often observed in any of the reports, and further development of a combined therapy is expected (Non Patent Literatures 3 and 4). The same applies to mesothelioma, and there are various results of a combined therapy of an anti-PD-1 antibody and an anti-CTLA-4 antibody (Non Patent Literature 5). As described above, various methods have been tried for combined therapy with an ICI, but it is known that it is difficult to obtain even an additive effect.

### Citation List

### Patent Literatures

Patent Literature 1: WO 2002/14462 A
Patent Literature 2: WO 2007/114876 A
Patent Literature 3: WO 2017/043613 A

### Non Patent Literatures

Non Patent Literature 1: Angevin, E., et al., Br J Cancer 116, 1126-1134 (2017)
Non Patent Literature 2: Takeda, M., et al., Lung Cancer 137, 64-70 (2019)
Non Patent Literature 3: Michael Boyer, et al., Journal of Clinical Oncology Published online January 29, 2021.DOI: 10.1200/JCO.20.03579 PMID: 33513313.
Non Patent Literature 4: Young Kwang Chae, et al., Journal for ImmunoTherapy of Cancer 6:39 (2018)
Non Patent Literature 5: Cornedine J. de Gooijer, et al., Frontiers in Oncology; 10: p. 187 (2020) DOI = 10.3389/fonc.2020.00187

### Summary of Invention

The present inventors have hitherto developed a humanized anti-CD26 antibody focusing on CD26 expressed in cancer cells as a treatment target molecule for cancer. In order to develop a new combined therapy that exhibits an antitumor effect for a longer period of time and provides a progression-free survival time as compared with administration of the humanized anti-CD26 antibody alone, studies have been made.

In order for an ICI to exhibit an antitumor effect, immune cells mainly including T cells are essential, and a cancer-bearing model in which a syngeneic mouse tumor cell line is transferred to a mouse is often used. Meanwhile, in order for the anti-CD26 antibody to exhibit an antitumor effect, a binding site on a human CD26 molecule is also important (Teruo Inamoto, et al., Clin Cancer Res; 13 (14): 4191-4200 (2007)), and a function of CD26 in an immune system is also largely different between a human and a mouse (Morimoto C, et al, Immunol Rev.; 161: 55-70 (1998)). Therefore, analysis in a human tumor cell line and a human immune system is essential for acquiring data of a humanized anti-CD26 antibody. Specifically, CD26 is also a T cell costimulatory molecule that transmits an activation signal to human T cells, and the humanized anti-CD26 antibody blocks binding between caveolin-1, which is a ligand of CD26, and CD26, that is, transmission of a CD26 costimulatory signal to T cells. Meanwhile, CD26 in mouse T cells does not function as a costimulatory molecule. In addition, regarding expression of CD26, CD26 in human T cells exhibits a triphasic pattern of strong positive, weak positive, and negative, whereas CD26 in mouse T cells is uniformly weakly positive. Regarding expression of CD26 in immune cells other than T cells, in humans, CD26 expression is observed in NKT cells other than T cells, but in B cells and NK cells, CD26 is hardly expressed, whereas in mice, also in B cells, CD26 exhibits weak positive equivalent to that in T cells. As described above, since there is a large difference in function in T cells, expression pattern in immune cells, and the like between a human and a mouse, analysis in a human immune system is essential in functional analysis of CD26 in an immune system. For the above reasons, first, an attempt was made to produce a human immunized mouse as a model animal for confirming a combined action of a humanized anti-CD26 antibody and an ICI. As a result, a model mouse excellent in generation of human T cells was successfully produced.

Subsequently, in a cancer-bearing model using this mouse, a combined effect of the humanized anti-CD26 antibody and the immune checkpoint inhibitor was studied. As a result, it has been found that the combined use of these has a stronger synergistic effect than each single drug. Such a synergistic effect could be confirmed for the first time by producing the model animal, and it is considered to reflect that a synergistic effect is similarly exhibited also in a human having expression of human CD26 in cancer cells and a human immune system (T cells) as in the model animal.

### Solution to Problem

A method for producing a model mouse of the present disclosure can be used for evaluation of molecules involved in a human immune system as a human immunized mouse in an animal model. In addition, a combined drug of the present disclosure contributes to achieving a more effective cancer treatment.

### Brief Description of Drawings

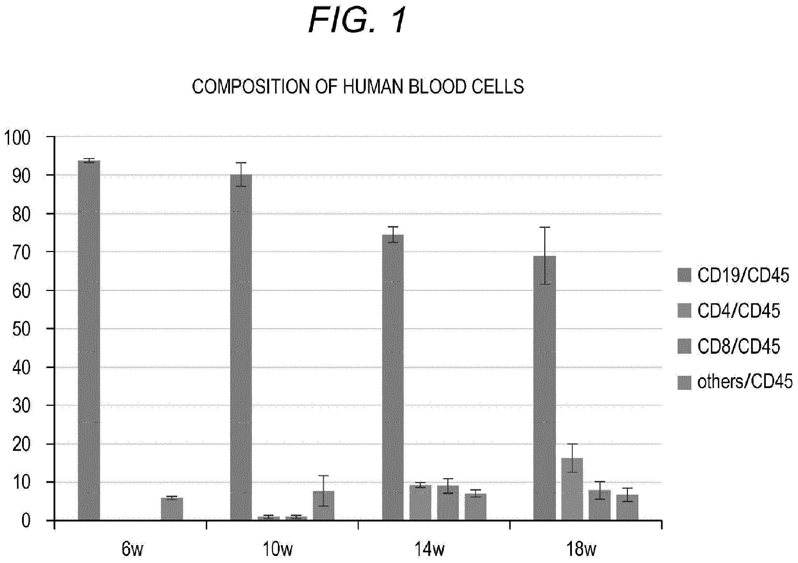

[Fig. 1] NOG mice were irradiated with radiation at a sublethal dose (100 cGy), and the next day, CD34 positive hematopoietic stem cells (1 × 10⁵) isolated and purified from human umbilical cord blood were transplanted into a tail vein of each of the mice. Six weeks, ten weeks, 14 weeks, and 18 weeks after transplantation of the human hematopoietic stem cells, 50 µl of blood was collected from the tail vein of each of the NOG mice, and a ratio of human blood cells in the blood of each of the mice was analyzed by flow cytometry (n = 10). Fig. 1 illustrates results thereof. The results are illustrated as mean value ± standard deviation of each individual.

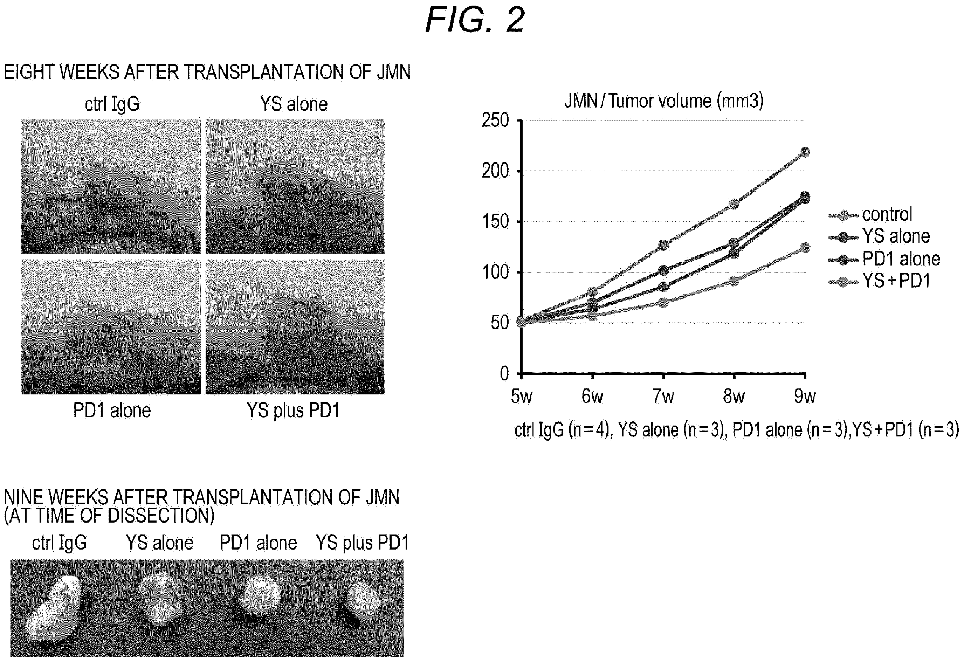

[Fig. 2] 13 weeks after transplantation of the human umbilical cord blood-derived CD34 positive hematopoietic stem cells into the tail veins of the NOG mice, generation and engraftment of human T cells in blood of the mice was confirmed, and then a human malignant mesothelioma cell line JMN (1 × 10⁶) was subcutaneously transplanted into a flank of each of the mice. Tumor formation could be observed four to five weeks after subcutaneous transplantation of JMN, and from a time point when five weeks had elapsed, administration of each of control human IgG1, a humanized anti-CD26 antibody (YS110) alone, mouse anti-human PD-1 mAb alone, and a combination of the humanized anti-CD26 antibody and the PD-1 antibody three times a week at 200 µg/dose was started, and continued until dissection in the ninth week. Tumor size measurement was performed continuously twice a week. The upper left drawing illustrates a representative example of an appearance photograph of a tumor size in each group at a time when eight weeks have elapsed after subcutaneous transplantation of JMN. The right drawing illustrates a graph of a temporal change of a tumor volume (average value inf each group) until dissection in the ninth week, and the lower left drawing illustrates a representative example of a tumor collected from the subcutaneous by dissection of the mice in the ninth week.

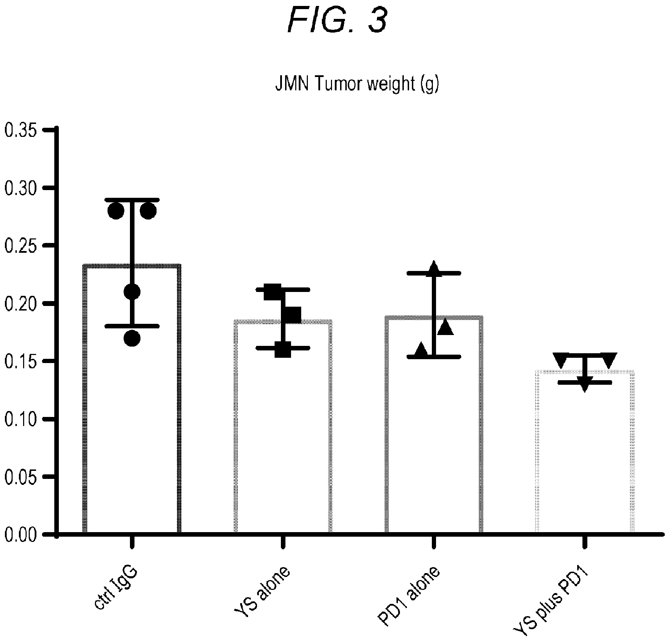

[Fig. 3] 13 weeks after transplantation of the human umbilical cord blood-derived CD34 positive hematopoietic stem cells into the tail veins of the NOG mice, a human malignant mesothelioma cell line JMN (1 × 10⁶) was subcutaneously transplanted into a flank of each of the mice. From a time point when five weeks had elapsed after subcutaneous transplantation of JMN, administration of each of control human IgG1, a humanized anti-CD26 antibody (YS110) alone, mouse anti-human PD-1 mAb alone, and a combination of the humanized anti-CD26 antibody and the PD-1 antibody three times a week at 200 µg/dose was started, and continued until dissection in the ninth week. Nine weeks after subcutaneous transplantation of JMN, each of the mice was dissected, and a subcutaneous tumor thereof was collected and weighed. Values of the measured tumor weights of the individuals are illustrated as mean ± standard deviation. Data are cumulative results of two independent experiments (control human IgG group n = 4, YS110 alone group n = 3, PD-1 antibody alone group n = 3, YS110 and PD-1 antibody combination group n = 3).

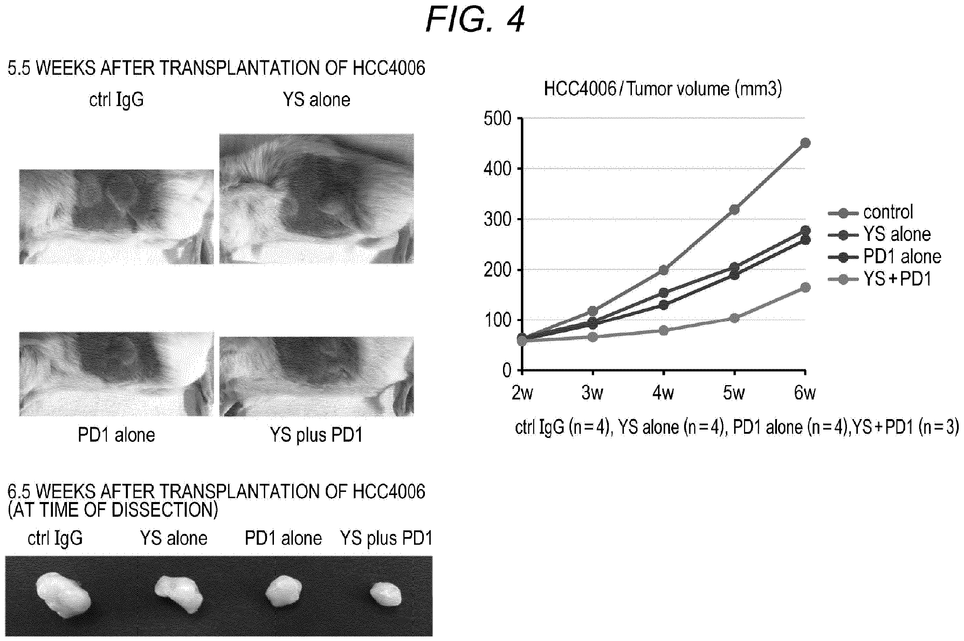

[Fig. 4] 14 weeks after transplantation of the human umbilical cord blood-derived CD34 positive hematopoietic stem cells into the tail veins of the NOG mice, generation and engraftment of human T cells in blood of the mice was confirmed, and then a human lung adenocarcinoma cell line HCC4006 (1 × 10⁶) was subcutaneously transplanted into a flank of each of the mice. Tumor formation could be observed two to three weeks after subcutaneous transplantation of HCC4006, and from a time point when three weeks had elapsed, administration of each of control human IgG1, a humanized anti-CD26 antibody (YS110) alone, mouse anti-human PD-1 mAb alone, and a combination of the humanized anti-CD26 antibody and the PD-1 antibody three times a week at 200 µg/dose was started, and continued until dissection in the 6.5th week. Tumor size measurement was performed continuously twice a week. The upper left drawing illustrates a representative example of an appearance photograph of a tumor size of each group at a time when 5.5 weeks have elapsed after subcutaneous transplantation of HCC4006. The right drawing illustrates a graph of a temporal change of a tumor volume (average value of each group) until dissection in the 6.5th week, and the lower left drawing illustrates a representative example of a tumor collected from the subcutaneous by dissection of each of the mice in the 6.5th week.

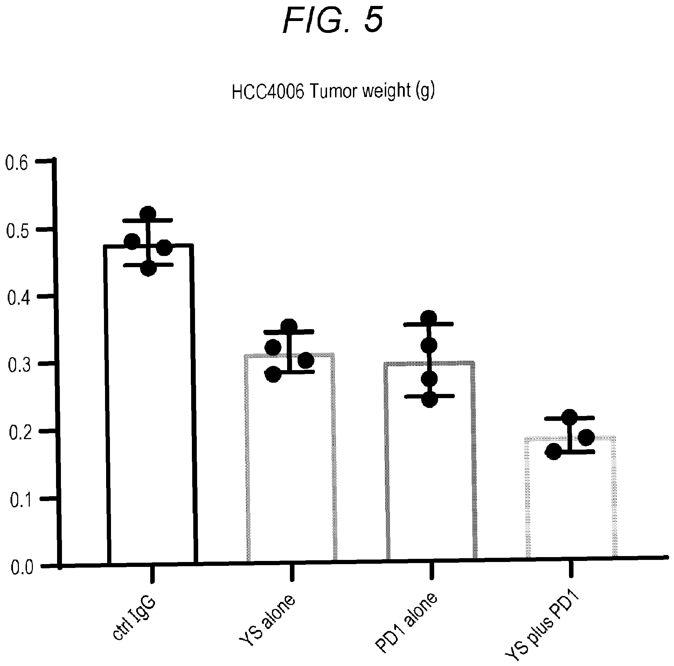

[Fig. 5] After 14 weeks from transplantation of the human umbilical cord blood-derived CD34 positive hematopoietic stem cells into the tail veins of the NOG mice, a human lung adenocarcinoma cell line HCC4006 (1 × 10⁶) was subcutaneously transplanted into a flank of each of the mice. From a time point when 3 weeks had elapsed after subcutaneous transplantation of HCC4006, administration of each of control human IgG1, a humanized anti-CD26 antibody (YS110) alone, mouse anti-human PD-1 mAb alone, and a combination of the humanized anti-CD26 antibody and the PD-1 antibody three times a week at 200 µg/dose was started, and continued until dissection in the 6.5th week. 6.5 weeks after subcutaneous transplantation of HCC4006, each of the mice was dissected, and a subcutaneous tumor thereof was collected and weighed. Values of the measured tumor weights of the individuals are illustrated as mean ± standard deviation. Data are cumulative results of two independent experiments (control human IgG group n = 4, YS110 alone group n = 4, PD-1 antibody alone group n = 4, YS110 and PD-1 antibody combination group n = 3).

### Description of Embodiments

### (Cancer)

Herein, "cancer", "cancer", and "tumor" are used interchangeably and mean a cell that has undergone malignant transformation so as to have pathogenicity for a host organism. Primary cancer cells (that is, cells obtained from the vicinity of a site of malignant transformation) can be easily distinguished from noncancerous cells by a well-established technique, particularly histological examination. Herein, definition of the cancer includes not only a primary cancer but also any cancer derived from the primary cancer, for example, a metastatic cancer. The cancer that appears as a solid tumor is detectable based on a tumor mass and can be identified, for example, by CAT scan, MR imaging, X-ray, ultrasound, or palpation, and/or detection of expression of one or more cancer-specific antigens in a sample obtained from a patient. The cancer may be, for example, a hematopoietic tumor which is a tumor of a blood cell or the like. Examples of the cancer herein include, but are not limited to, lung cancer, non-small cell lung cancer, small cell lung cancer, non-Hodgkin's lymphoma, adrenocortical cancer, AIDS-related cancer, AIDS-related lymphoma, pediatric cerebellar astrocytoma, pediatric cerebral astrocytoma, basal cell cancer, skin cancer (non-melanoma), biliary tract cancer, extrahepatic bile duct cancer, intrahepatic bile duct cancer, bladder cancer, cancer of bone or joint, osteosarcoma and malignant fibrous histiocytoma, brain cancer, brain tumor, brain stem glioma, cerebellar astrocytoma, glioma, cerebral astrocytoma/malignant glioma, ependymoma, medulloblastoma, supratentorial primitive neuroectodermal tumor, visual pathway and hypothalamic glioma, head and neck cancer, metastatic squamous cell carcinoma, bronchial adenoma/carcinoid, carcinoid tumor, nervous system lymphoma, cancer of central nervous system, central nervous system lymphoma, neuroblastoma, pediatric syndrome, Seziary syndrome, extracranial germ cell tumor, extragonadal germ cell tumor, extrahepatic bile duct cancer, cancer of eye, intraocular melanoma, retinoblastoma, salivary gland cancer, oral cancer, oral cavity cancer, oropharyngeal cancer, oral cancer, cancer of tongue, esophageal cancer, gastric cancer, gastrointestinal carcinoid tumor, gastrointestinal stromal tumor (GIST), small intestine cancer, colon cancer, colorectal cancer, rectal cancer, anorectal cancer, anal cancer, germ cell tumor, hepatocyte (liver) cancer, Hodgkin lymphoma, laryngeal cancer, pharyngeal cancer, hypopharyngeal cancer, nasopharyngeal cancer, sinus and nasal cavity cancer, throat cancer, pancreatic islet cell tumor (endocrine pancreas), pancreatic cancer, parathyroid cancer, liver cancer, gallbladder cancer, appendix cancer, kidney cancer, urethral cancer, transitional cell carcinoma of renal pelvis and ureter and other urinary cancers, squamous cell carcinoma, penile cancer, testicular cancer, thymoma, thymoma and thymic carcinoma, thyroid cancer, prostate cancer, ovarian cancer, ovarian epithelial cancer, ovarian low malignancy tumor, ovarian germ cell tumor, gestational choriocarcinoma, breast cancer, uterine cancer, uterine sarcoma, cervical cancer, endometrial cancer, uterine cancer, vaginal cancer, vulvar cancer, Wilms' tumor, acute lymphoblastic leukemia, acute myelogenous leukemia, chronic lymphocytic leukemia, chronic myelogenous leukemia, multiple myeloma, chronic myeloproliferative disease, hairy cell leukemia, primary central nervous system lymphoma, chronic myeloproliferative disease, cutaneous T cell lymphoma, lymphoid neoplasm, macroglobulinemia of Waldenstram, medulloblastoma, skin cancer (non-melanoma), skin cancer (melanoma), brown tumor, Merkel cell skin cancer, mesothelioma, malignant mesothelioma, multiple endocrine tumor syndrome, myelodysplastic syndrome, myelodysplastic/myeloproliferative disease, pineoblastoma and pituitary tumor, plasmacytoma, pleuropulmonaryblastoma, retinoblastoma, rhabdomyosarcoma, sarcoma, Ewing's tumor family, soft tissue sarcoma, soft tissue sarcoma, mycosis fungoides, and Kaposi's sarcoma.

Herein, cancer or cancer cells are mammalian cancer or mammalian cancer cells, and preferably human cancer or human cancer cells. The cancer herein may be cancer expressing CD26.

### (Antibody)

Herein, an "antibody" is an immunoglobulin molecule capable of specifically binding to a target such as a carbohydrate, a polynucleotide, a lipid, or an antibody via at least one antigen recognition site located in a variable region of the immunoglobulin molecule. As used herein, the term antibody includes not only a whole polyclonal antibody and a whole monoclonal antibody but also fragments thereof. It is known that a variable region (particularly CDRs) of an antibody imparts a binding property, and it is widely known to those skilled in the art that even an antibody fragment that is not a whole antibody can use the binding property. Herein, a "fragment" or an "antigen-binding fragment" of an antibody means a protein or a peptide containing a part (partial fragment) of the antibody and retaining action (immunoreactivity/binding property) of the antibody on an antigen. Examples of such an immunoreactive fragment include F(ab')₂, Fab', Fab, Fabs, single chain Fv (hereinafter, referred to as "scFv"), (tandem) bi-specific single chain Fv (sc(Fv)₂), single chain triple body, nanobody, divalent VHH, pentavalent VHH, minibody, (double chain) diabody, tandem diabody, bi-specific tribody, bi-specific bibody, dual affinity targeting molecule (DART), triabody (or tribody), tetrabody (or [sc(Fv)₂]₂ or (scFv-SA)₄), disulfide-binding Fv (hereinafter, referred to as "dsFv"), compact IgG, polymer chain antibody, and polymers thereof (See Nature Biotechnology, 29(1): 5-6 (2011); Maneesh Jain et al., TRENDS in Biotechnology, 25(7) (2007): 307-316; and Christoph stein et al., Antibodies (1): 88-123 (2012)). Herein, the immunoreactive fragment may be any of mono-specific, bi-specific, tri-specific, and multispecific. The fragment of the antibody may include a fragment having a length of at least about 10 amino acids, at least about 25 amino acids, at least about 50 amino acids, at least about 75 amino acids, or at least about 100 amino acids.

The antibody includes any class of antibody such as IgG, IgA, or IgM (or subclasses thereof), and does not need to belong to a particular class. Immunoglobulins are classified into different classes according to an antibody amino acid sequence of a constant domain of a heavy chain. There are five main immunoglobulin classes: IgA, IgD, IgE, IgG, and IgM, some of which may be further classified into subclasses (isotypes) of, for example, IgG1, IgG2, IgG3, IgG4, IgA1, and IgA2. Constant domains of corresponding heavy chains of immunoglobulins belonging to different classes are referred to as α, 5, ε, γ, and µ. Subunit structures and three-dimensional structures for immunoglobulins belonging to different classes are well known. Preferably, the antibody may be an IgG antibody, particularly an IgG1 antibody or an IgG2 antibody, and may be a human IgG antibody.

The antibody herein can be a monoclonal antibody or a polyclonal antibody, and is preferably a monoclonal antibody. Herein, the term "monoclonal antibody" refers to an antibody obtained from a cell population that produces a substantially homogeneous antibody. That is, the individual antibodies contained in the cell population are the same except for natural mutants that may be slightly present. The monoclonal antibody is directed against a single antigen site and is highly specific. Furthermore, in contrast to a typical polyclonal antibody containing different antigens targeting different determinants (epitopes), each monoclonal antibody targets a single determinant of an antigen. The modifier "monoclonal" indicates a property of an antibody obtained from a substantially homogeneous population of antibody-producing cells, and is not to be understood as requiring production of an antibody by a particular method. For example, a monoclonal antibody used according to the present disclosure may be prepared by a recombinant DNA method as described in U.S. Patent No. 4,816,567. The monoclonal antibody can also be isolated, for example, from a phage library prepared using a technique described in McCafferty et al., Nature, 348: 552-554 (1990).

The antibody herein can be a chimeric antibody, a humanized antibody, a fully human antibody, or an animal antibody adapted to any animal to be treated other than human, and is preferably a humanized antibody. As used herein, the "humanized" antibody is an antibody including a minimal sequence derived from a non-human immunoglobulin and a sequence derived from human or an antigen-binding fragment thereof (Fv, Fab, Fab', F(ab')₂, another antigen-binding partial subsequence of the antibody, or the like). Some the humanized antibodies are human immunoglobulins (recipient antibodies) in which a residue derived from a complementarity determining region (CDR) of a receptor is replaced with a CDR-derived residue of a non-human species (donor antibody) such as a mouse, a rat, or a rabbit, having desired specificity, affinity, and ability. An Fv framework region (FR) residue of a human immunoglobulin may be replaced with a corresponding non-human-derived residue. The humanized antibody includes a variable region derived from a non-human species (donor antibody) such as a mouse, a rat, or a rabbit, having desired specificity, affinity, and/or ability, and one or more residues in the Fv framework region and/or one or more CDR residues may be replaced with corresponding human residues (that is, residues derived from a human antibody sequence). The humanized antibody can include a residue that is not present in a transferred original CDR or a framework sequence for further improvement and optimization of antibody performance. The humanized antibody most preferably includes at least a part of a constant region (Fc) or a domain (generally a human immunoglobulin) of a human immunoglobulin. Some of the humanized antibodies have a modified Fc region as described in WO 99/58572 A. Some forms of the humanized antibody have one or more (for example, 1, 2, 3, 4, 5, or 6) CDRs changed relative to an original antibody, and the CDRs are also referred to as one or more CDRs "derived from" one or more CDRs of the original antibody.

In a certain embodiment, the antibody includes one or more constant regions, for example, human constant regions. The constant region can be a constant region of a heavy chain and/or a constant region of a light chain. The antibody may include a constant region having at least about 80%, at least about 85%, at least about 90%, at least about 95%, at least about 98%, or 100% identity to the human constant region. The antibody may include an Fc region, for example, a human Fc region. The antibody may include an Fc region having at least about 80%, at least about 85%, at least about 90%, at least about 95%, at least about 98%, or 100% identity to the human Fc region.

The antibody used herein specifically binds to a target molecule (CD26 or an immune checkpoint molecule, also referred to as "antigen" herein throughout). Binding affinity of an antibody for an antigen as used herein includes affinity having a dissociation constant (that is, Kd) of less than 1 × 10⁻⁵ M, less than 5 × 10⁻⁵ M, less than 1 × 10⁻⁶ M, less than 5 × 10⁻⁷ M, less than 1 × 10⁻⁷ M, less than 5 × 10⁻⁸ M, less than 1 × 10⁻⁸ M, less than 5 × 10⁻⁹ M, less than 1 × 10⁻⁹ M, less than 5 × 10⁻¹⁰ M, less than 1 × 10⁻¹⁰ M, less than 5 × 10⁻¹¹ M, or less than 1 × 10⁻¹¹ M. The dissociation constant may also be 1 × 10⁻¹⁵ M or more, 5 × 10⁻¹⁵ M or more, 1 × 10⁻¹⁴ M or more, 5 × 10⁻¹⁴ M or more, 1 × 10⁻¹³ M or more, 5 × 10⁻¹³ M or more, 1 × 10⁻¹² M or more, 5 × 10⁻¹² M or more, 1 × 10⁻¹¹ M or more, 5 × 10⁻¹¹ M or more, 1 × 10⁻¹⁰ M or more, or 5 × 10⁻¹⁰ M or more. A method for determining affinity is known in the present technical field. For example, the binding affinity may be determined using a BIAcore biosensor, a KinExA biosensor, a scintillation proximity assay, ELISA, ORIGEN immunoassay (IGEN), fluorescence quenching, fluorescence transfer, and/or yeast display. The affinity may also be screened using an appropriate bioassay.

One method for determining binding affinity of an antibody for an antigen is to measure affinity of a monofunctional Fab fragment of an antibody. In order to obtain the monofunctional Fab fragment, an antibody, for example, IgG, is cleaved with papain or can be expressed by a recombinant technique. Affinity of a Fab fragment of a monoclonal antibody can be determined by a surface plasmon resonance (SPR) system (BIAcore 3000 (trademark), BIAcore, Piscaway, NJ). An SA chip (streptavidin) is used according to supplier's instructions. A biotinylated antigen is diluted in HBS-EP (100 mM HEPES pH 7.4, 150 mM NaCl, 3 mM EDTA, 0.005% P20) and can be injected onto the chip at a concentration of 0.005 mg/mL. Using variable flow times across individual chip channels, two ranges of antigen density are achieved: 10 to 20 response units (RU) for detailed kinetic testing and 500 to 600 RU for concentration. A mixture of a Pierce elution buffer and 4 M NaCl (2 : 1) efficiently removes binding Fab while maintaining activity of CD26 on the chip for 200 or more injections. An HBS-EP buffer can be used for all BIAcore assays as a running buffer. A step-wise diluted solution of a purified Fab sample (0.1 to 10 x estimated KD) is injected at 100 µL/min for two minutes, and dissociation time up to 30 min is usually tolerated. The concentration of Fab protein can be determined by ELISA and/or SDS-PAGE electrophoresis using standard Fab having a known concentration (determined by amino acid analysis). A binding rate (kon) and a dissociation rate (koff) as reaction rates are simultaneously obtained by fitting data to a 1 : 1 Langmuir binding model (Lofas & Johnsson, 1990) using a BIAevaluation program. An equilibrium dissociation constant (KD) value is calculated as koff/kon.

The antibody described herein may be modified, examples of such a modification include a functionally equivalent antibody and a variant with enhanced or attenuated activity that do not significantly affect the properties of the antibody. The modification of the antibody is a routine operation in the present technical field and does not need to be described in detail herein. Examples of the modification include a conservative substitution of an amino acid residue, insertion, addition, or deletion of one or more amino acids that do not significantly degrade functional activity, and use of a chemical analog. Insertion or addition of an amino acid sequence includes addition of an amino acid to an amino terminal and/or a carboxyl terminal, and insertion of a single or a plurality of amino acid residues into a sequence. Examples of the insertion into a terminal include an antibody with an N-terminal methionyl residue and an antibody fused to an epitope tag. Other insertion variants of an antibody molecule include fusion of an enzyme or an antibody that extends a serum half-life of the antibody to an N-terminal or a C-terminal of the antibody.

In a substitution variant, at least one amino acid residue of an antibody sequence is removed, and a different residue is inserted into this position. A site where the most effect is obtained by substitutional mutagenesis includes a CDR, but an FR is also intended to be changed. The conservative substitution is presented in Table 1. When such a substitution changes biological activity, the substitution may be named "exemplary substitution" in Table 1, or furthermore, regarding an amino acid class, a more substantial change may be introduced and a product may be screened as described below.

**[Table 1]**

| Original residue | Conservative substitution | Exemplary substitution |
|---|---|---|
| Ala (A) | Val | Val; Leu; Ile |
| Arg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn; Glu |
| Glu (E) | Asp | Asp; Gin |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

A substantial modification in biological properties of an antibody are achieved by selecting a substitution that significantly changes an effect of the modification on (a) a structure of an antibody main chain in a substitution region, such as a sheet or helix conformation, (b) a charge or hydrophobicity of a molecule at a target site, or (c) maintenance of a volume of a side chain. Residues present in a natural state are classified into the following groups based on common side chain properties:
(1) Hydrophobicity: Norleucine, Met, Ala, Val, Leu, and Ile;
(2) Neutral hydrophilicity: Cys, Ser, and Thr;
(3) Acidity: Asp and Glu;
(4) Basicity: Asn, Gln, His, Lys, and Arg;
(5) Residues affecting chain orientation: Gly and Pro; and
(6) Aromatic: Trp, Tyr, and Phe.

A non-conservative substitution is made by exchanging one member in these classes for a member in another class. A more conservative substitution includes exchanging one member in a certain class for another member in the same class. An amino acid substitution in a variable region can change binding affinity and/or specificity. For example, 1 to 5 conservative amino acid substitutions are made within a CDR domain. For example, 1 to 3 conservative amino acid substitutions are made within a CDR3 domain.

Substitution of any cysteine residue not involved in maintaining an appropriate conformation of the antibody (usually replaced with serine) can improve oxidative stability of a molecule and prevent abnormal crosslinking. Conversely, particularly when the antibody is an antibody fragment such as an Fv fragment, stability of the antibody can be improved by adding a cysteine bond to the antibody.

The antibody described herein also includes a glycosylated antibody and an aglycosylated antibody, and an antibody with another post-translational modification, such as glycosylation, acetylation, or phosphorylation in a different sugar. The antibody is glycosylated at a conserved position in a constant region thereof (Jefferis and Lund, 1997, Chem. Immunol. 65: 111-128; Wright and Morrison, 1997, TibTECH 15: 26-32). Absence of fucose in an Fc region of the antibody has been reported to affect antibody-dependent cellular cytotoxicity (ADCC).

### (Anti-CD26 antibody)

In a certain embodiment, an anti-CD26 antibody of the present disclosure includes both a heavy chain variable region including an amino acid sequence having at least about 80% identity to an amino acid sequence selected from the group consisting of SEQ ID NOs: 8 to 14 and a light chain variable region including an amino acid sequence having at least about 80% identity to an amino acid sequence selected from the group consisting of SEQ ID NOs: 1 to 7.

Table 2 presents amino acid sequences of humanized VL variants that are X376 (SEQ ID NO: 1), X377 (SEQ ID NO: 2), X378 (SEQ ID NO: 3), X379 (SEQ ID NO: 4), X380 (SEQ ID NO: 5), X381 (SEQ ID NO: 6), and X394 (SEQ ID NO: 7). The schemes with Kabat numbers and SEQ ID numbers have the same light chain variable region.

**[Table 2]**

| Sequential Numbering | 10 | 20 | 30 | 40 | 50 | 60 | 70 | 80 | 90 | 100 |
|---|---|---|---|---|---|---|---|---|---|---|
| | 1234567890123456789012345678901234567890123456789012345678901234567890123456789012345678901234567890123456789012345678901234567 <------------FR1-------------><---CDR1----><------FR2------><CDR2-><--------------FR3--------------><-CDR3---><--FR4---> | | | | | | | | | |
| CMO3 VL | DIQMTQSPSSLSASLGDRVTITCSASQGIRNSLNWYQQKPDGAVKLLIYYSSNLHSGVPSRFSGSGSGTDFSLTISNLEPEDIATYYCQQSIKLPFTFGSGTKLEIK DILMTQSPSSLSASPGDRVTISCRASQDIRNNLNWYQQKPGQAPRLLIYYSSNLESGVPDRFSGSGSGTDFTLTISRLEPEDFAAYYCQQSIKLPLTFGSGTKVEIK EIELTQSPSSLSVSLGDRVTISCSASQDIRNNLNWYQQKPGQAPRLLIYYSSNLQTGVPARFSGSGSGTDFTLTISRLEPEDVAAYYCQQSIKLPFTFGSGTKVEIK DIEMTQSPSSLSASAGERVTISCRASQGIRNSLNWYQQKPGQAPRLLIYYSSNLQTGVPSRFSGSGSGTDFTLTISRLQAEDFATYYCQQSNKLPFTFGSGTKVEIK DILLTQSPSSLSATPGERATITCRASQGIRNNLNWYQQKPGQAPRLLIYYSSNLQSGVPSRFSGSGSGTDFTLTISRLQPEDVAAYYCQQSIKLPFTFGSGTKVEIK EIEMTQSPSSLSVSAGERATISCSASQDIRNSLNWYQQKPGQAPRLLIYYSSNLHTGVPARFSGSGSGTDFTLTISRLEPEDVATYYCQQSNKLPLTFGSGTKVEIK EIELTQSPSSLSVSPGDRVTISCSASQGIRNSLNWYQQKPGQAPRLLIYYSSNLHTGVPARFSGSGSGTDFTLTISRLQAEDFATYYCQQSIKLPLTFGSGTKVEIK DILMTQSPSSLSASPGDRVTISCRASQDIRNNLNWYQQKPGQAPRLLIYYSSNLQTGVPARFSGSGSGTDFTLTISRLEPEDFAAYYCQQSIKLPLTFGSGTKVEIK | | | | | | | | | |
| X376 | | | | | | | | | | |
| X377 | | | | | | | | | | |
| X378 | | | | | | | | | | |
| X379 | | | | | | | | | | |
| X380 | | | | | | | | | | |
| X381 | | | | | | | | | | |
| X394 | | | | | | | | | | |
| Kabat Numbering | | 24 | 34 | | 50 | 56 | | | 89 | 97 |
| (same as sequential numbering; no Insertion) | | | | | | | | | | |

Table 3 presents amino acid sequences of humanized VH variants that are X384 (SEQ ID NO: 8), X385 (SEQ ID NO: 9), X386 (SEQ ID NO: 10), X387 (SEQ ID NO: 11), X388 (SEQ ID NO: 12), X399 (SEQ ID NO: 13) and X420 (SEQ ID NO: 14). Table 3 presents both a sequential numbering scheme and a Kabat numbering scheme. The Kabat numbering scheme includes 82a, 82b, and 82c.

**[Table 3]**

| Sequential Numbering | 10 | 20 | 30 | 40 | 50 | 60 | 70 | 80 | 90 | 100 | 110 |
|---|---|---|---|---|---|---|---|---|---|---|---|
| | 1234567890123456789012345678901234567890123456789012345678901234567890123456789012345678901234567890123456789012345678901234567890123456 <-----------FR1----------><--CDR1--><----FR2-----><-----CDR2------><-------------------FR3-------------------><-CDR3-><--FR4---> | | | | | | | | | | |
| CMO3 VB | QVKLQESGPGLVQPSQTLSITCTVSGFSLTTYGVHWVRQSPGKGLEWLGVIWGGGRTDYDAAFISRLSISKDNSKSQVFFKMNSLQANDTAIYYCVRNRHDWFDYWGQGTTVTVSS EVQLVESGAGVKQPGGTLRLTCTASGFSLTTYGVHWVRQAPGKGLEWVGVIWGDGRTDYDAAFMSRVTISKDTSKSTVYLQMNSLRAEDTAVYYCMRNRHDWFDYWGQGTTVTVSS EVQLVQSGGGVKQPGETLRLTCTASGFSLTTYGVHWVRQAPGKGLEWVGVIWGDGRTDYDAAFMSRVTISKDTSKSTAYLQMNSLRAEDTAVYYCMRNRHDWFDYWGQGTTVTVSS EVQLVESGAGVEQPGGTLRLTCTASGFSLSTYGVHWVRQAPGKGLEWMGVIWGDGRTDYDAAFMSRVTISRDTSKSTAYLQLNSLRAEDTAVYYCVRNRHDWFDYWGQGTTVTVSS EVQLVESGAELVQPGGSLRLTCKASGFTINTYGVHWVRQAPGKGLEWMGVIWGGGRTDYDASFMSRVTISKDNSKNTAYLQLNSLRAEDTAVYYCTRSRHDWFDYWGQGTTVTVSS EVQLVQSGGGLKQPGETLRLSCTASGYSLTTYGVHWVRQAPGKGLEWMGVIWGDGRTDYDSSFMSRVTISKDTSKSTAYLQLNSLRAEDTAVYYCTRNRHDWFDYWGQGTTVTVSS EVQLVQSGGGVKQPGETLRLTCTASGFSLSTYGVHWVRQAPGKGLEWVGVIWGDGRTDYDAAFMSRVTISKDTSKSTVYLQMNSLRAEDTAVYYCMRNRHDWFDYWGQGTTVTVSS EVQLVESGGGVKQPGETLRLTCTASGFSLSTYGVHWVRQAPGKGLEWVGVIWGDGRTDYDAAFMSRVTISKDTSKSTVYLQMNSLRAEDTAVYYCMRNRHDWFDYWGQGTTVTVSS | | | | | | | | | | |
| X384 | | | | | | | | | | | |
| X385 | | | | | | | | | | | |
| X386 | | | | | | | | | | | |
| X367 | | | | | | | | | | | |
| X388 | | | | | | | | | | | |
| X399 | | | | | | | | | | | |
| X420 | | | | | | | | | | | |
| Kabat Numbering | | 26 | 35 | | 50 | 65 | | abc3456789012345678901234567890123 | | | |
| | | | | | | | | 82 | 90 | 100 | 110 |
| | | | | | | | | | 95 | 102 | |

For example, the anti-CD26 antibody has the heavy chain variable region set forth in SEQ ID NO: 8 and the light chain variable region set forth in SEQ ID NO: 4. Alternatively, the anti-CD26 antibody has a heavy chain including SEQ ID NO: 17 or a fragment thereof, preferably a heavy chain including SEQ ID NO: 17. In a certain embodiment, a heavy chain of the anti-CD26 antibody has a sequence obtained by removing a signal sequence from SEQ ID NO: 17. In a certain embodiment, a heavy chain of the anti-CD26 antibody includes a variable region of SEQ ID NO: 17.

### Heavy chain (SEQ ID NO: 17) (underlined parts indicate a signal sequence)

MEWSWVFLFFLSVTTGVHSEVQLVESGAGVKQPGGTLRLTCTASGFSLTTYGVHWVRQA

PGKGLEWVGVIWGDGRTDYDAAFMSRVTISKDTSKSTVYLQMNSLRAEDTAVYYCMRNR

HDWFDYWGQGTTVTVSSASTKGPSVFPLAPSSKSTSGGTAALGCLVKDYFPEPVTVSWN

SGALTSGVHTFPAVLQSSGLYSLSSVVTVPSSSLGTQTYICNVNHKPSNTKVDKKVEPK

SCDKTHTCPPCPAPELLGGPSVFLFPPKPKDTLMISRTPEVTCVVVDVSHEDPEVKFNW

YVDGVEVHNAKTKPREEQYNSTYRVVSVLTVLHQDWLNGKEYKCKVSNKALPAPIEKTI

SKAKGQPREPQVYTLPPSRDELTKNQVSLTCLVKGFYPSDIAVEWESNGQPENNYKTTP

PVLDSDGSFFLYSKLTVDKSRWQQGNVFSCSVMHEALHNHYTQKSLSLSPGK

For example, the anti-CD26 antibody has a light chain including SEQ ID NO: 18 or a fragment thereof, and preferably has a light chain including SEQ ID NO: 18. In a certain embodiment, a light chain of the anti-CD26 antibody has a sequence obtained by removing a signal sequence from SEQ ID NO: 18. In a certain embodiment, a light chain of the anti-CD26 antibody includes a variable region of SEQ ID NO: 18.

### Light chain (SEQ ID NO: 18) (underlined parts indicate a signal sequence)

MSVPTQVLGLLLLWLTDARCDILLTQSPSSLSATPGERATITCRASQGIRNNLNWYQQK

PGQAPRLLIYYSSNLQSGVPSRFSGSGSGTDFTLTISRLQPEDVAAYYCQQSIKLPFTF

GSGTKVEIKRTVAAPSVFIFPPSDEQLKSGTASVVCLLNNFYPREAKVQWKVDNALQSG

NSQESVTEQDSKDSTYSLSSTLTLSKADYEKHKVYACEVTHQGLSSPVTKSFNRGEC

Herein, "YS110" which is a humanized anti-CD26 antibody means an antibody whose heavy chain consists of the amino acid sequence set forth in SEQ ID NO: 17 and whose light chain consists of the amino acid sequence set forth in SEQ ID NO: 18.

### (Immune checkpoint inhibitor)

The immune checkpoint inhibitor means a drug that binds to an immune checkpoint molecule to inhibit a function of the immune checkpoint molecule. Examples of the function of the immune checkpoint molecule include a function of suppressing an immune response against self in order to maintain immune homeostasis and a function of suppressing an excessive immune response. Examples of the immune checkpoint molecule include PD-1 or a ligand thereof (PD-L1 or PD-L2), CTLA-4 or a ligand thereof (B7 (CD80/86)), TIM-3 or a ligand thereof (Galectin -9), BTLA or a ligand thereof (HVEM), LAG-3, TCR or a ligand thereof (MHC-II). The immune checkpoint inhibitor may be an antibody or an antigen-binding fragment thereof, an aptamer, or a small molecule compound as long as it can bind to these immune checkpoint molecules to inhibit functions of the immune checkpoint molecules, but is preferably an antibody or an antigen-binding fragment thereof. As the antibody that binds to the immune checkpoint molecule, for example, an anti-CTLA-4 antibody (ipilimumab), an anti-PD-1 antibody (pembrolizumab or nivolumab), and an anti-PD-L1 antibody (atezolizumab or durvalumab) are commercially available as pharmaceuticals for a human cancer treatment.

As used herein, "treatment" is an approach for obtaining a beneficial or desired clinical result. In the present disclosure, the beneficial or desired clinical result includes, but are not limited to, alleviation of one or more symptoms, diminishment of extent of disease, stabilized (that is, non-deteriorated) state of disease, delay or slowing of disease progression, amelioration or palliation of a disease state, and remission (partial or total), regardless of being detectable or undetectable. "Treatment" may also mean prolonging life expectancy relative to expected life expectancy if the treatment is not received.

An "effective amount" is an amount sufficient to achieve a beneficial or desired clinical result including a clinical result. The effective amount can be administered in one or more administrations. In the present disclosure, an effective amount of an anti-CD26 antibody or an immune checkpoint inhibitor described herein is an amount sufficient to delay progression of a state related to tumor growth or reduce a tumor. The effective amount may vary or depend on other factors such as patient's medical history and the type (and/or amount) of an anti-CD26 antibody or an immune checkpoint inhibitor used.

A pharmaceutical composition herein may optionally contain a pharmaceutically acceptable additive. "Pharmaceutically acceptable additive" contains any material that makes it possible for an active ingredient to maintain biological activity when combined with the active ingredient, is non-reactive with a subject's immune system when delivered, and is non-toxic to the subject. Examples thereof include, but are not limited to, any standard pharmaceutical carrier such as phosphate buffered saline, water, an emulsion such as an oil/water emulsion, and various types of wetting agents. A preferred diluent for spray or parenteral administration is phosphate buffered saline or physiological saline (0.9%). A composition containing such a carrier is formulated by a well-known conventional method (See, for example, Remington's Pharmaceutical Sciences, 18th edition, edited by A. Gennaro, Mack Publishing Co., Easton, PA, 1990 and Remington, The Science and Practice of Pharmacy 20th edition, Mack Publishing, 2000).

In a certain embodiment, the antibody is expressed in any organism including bacteria, yeast, plants, insects, and mammals without being limited thereto, or a host cell derived from any organism. Examples of a specific type of cells include, but are not limited to, Drosophila melanogaster cells, Saccharomyces cerevisiae and other yeasts, E. coli, Bacillus subtilis, SF9 cells, HEK-293 cells, Neurospora, BHK cells, CHO cells, COS cells, Hela cells, fibroblasts, Schwann cell lines, immortalized mammalian bone marrow and lymphoid cell lines, Jurkat cells, mast cells and other endocrine and exocrine cells, and neuronal cells.

Various protein expression systems, vectors, and cell media useful for production of the antibody are known to those skilled in the art. See, for example, WO 03/054172 A, WO 04/009823 A, and WO 03/064630 A (the entirety of which is incorporated herein by reference). In a certain embodiment, a glutamine synthase (GS) expression system is used for expression of the antibody.

Examples of a subject to be treated include mammals such as human, bovine, equine, canine, feline, porcine, and ovine, and human is preferable.

The pharmaceutical composition of the present disclosure may adopt any oral or parenteral formulation as long as it is a formulation that can be administered to a patient. Examples of a composition for parenteral administration include an injection and a nasal drop. An injection is preferable. Examples of a dosage form of the pharmaceutical composition of the present disclosure include a liquid agent and a lyophilized formulation. When the pharmaceutical composition of the present disclosure is used as an injection, an additive, for example, a solubilizing agent such as propylene glycol or ethylenediamine, a buffering agent such as a phosphate, an isotonizing agent such as sodium chloride or glycerin, a stabilizer such as a sulfite, a preservative such as phenol, or a soothing agent such as lidocaine (See "Pharmaceutical additive dictionary", Yakuji Nippo Limited. and "Handbook of Pharmaceutical Excipients Fifth Edition", APhA Publications) can be added as necessary. In addition, when the pharmaceutical composition of the present disclosure is used as an injection, examples of a storage container include an ampoule, a vial, a prefilled syringe, a pen-type syringe cartridge, and a drip bag.

A combined drug of the present disclosure may be provided as one formulation containing all or some of two or more types of drugs to be used in combination, or may be provided as a formulation containing each drug intended for combined use alone. Alternatively, the combined drug of the present disclosure may be provided as a kit or a set for combined use including a formulation containing each drug to be used in combination alone.

In another aspect, the present disclosure relates to a method for treating cancer, the method including administering an anti-CD26 antibody and an immune checkpoint inhibitor to a patient having cancer. In another aspect, the present disclosure relates to a pharmaceutical composition containing an anti-CD26 antibody and an immune checkpoint inhibitor used for treating cancer. Alternatively, the present disclosure relates to use of an anti-CD26 antibody and an immune checkpoint inhibitor for producing a cancer treatment agent. In a cancer treatment described herein, administration of an anti-CD26 antibody and administration of an immune checkpoint inhibitor in a cancer patient do not need to be performed in this order, and either of these may be performed first. That is, an immune checkpoint inhibitor may be administered after administration of an anti-CD26 antibody, the anti-CD26 antibody may be administered after administration of the immune checkpoint inhibitor, or both may be administered simultaneously or continuously.

The pharmaceutical composition of the present disclosure may be administered to a mammal by injection (for example, systemically, intravenously, intraperitoneally, subcutaneously, intramuscularly, intraportally, or into a cancer tissue) or by another method that ensures delivery to a bloodstream in an effective form (for example, injection).

A method (including a therapy) described herein may be performed by a single direct injection at a single time point or multiple time points to a single site or multiple sites. Administration may be administered to multiple sites almost simultaneously. An administration frequency is determined and adjusted in a course of treatment, and is based on a result desired to be achieved. In some cases, a sustained release formulation of the pharmaceutical compositions of the present disclosure may be suitable. Various formulations and devices for achieving sustained release are well known in the present field.

An effective dose and schedule for administering the pharmaceutical composition of the present disclosure are empirically determined, and such a determination method is within common general technical knowledge of the present technical field. Those skilled in the art will understand that a dose of the pharmaceutical composition of the present disclosure to be administered will vary depending on, for example, a mammal to be inoculated with the pharmaceutical composition of the present disclosure, a route of administration, the specific type of a drug to be used, and other drugs to be administered to the mammal. A typical dosage per day of the pharmaceutical composition of the present disclosure may range from about 1 µg/kg body weight to 100 mg/kg body weight or more per day, depending on the above-described factors. In general, any of the following dosages may be used: at least about 50 mg/kg body weight; at least about 10 mg/kg body weight; at least about 3 mg/kg body weight; at least about 1 mg/kg body weight; at least about 750 µg/kg body weight; at least about 500 µg/kg body weight; at least about 250 µg/kg body weight; at least about 100 µg/kg body weight; at least about 50 µg/kg body weight; at least about 10 µg/kg body weight; and at least about 1 µg/kg body weight, or a dosage more than this is administered.

Hereinafter, Examples will be described in order to describe the present disclosure in more detail, but the present disclosure is not limited thereto. Note that all documents cited throughout this application are incorporated herein by reference as they are.

### (Material and method)

### 1) Cells

Human malignant mesothelioma cell line JMN and human pulmonary adenocarcinoma cell line HCC 4006 were cultured in an RPMI 1640 medium to which 10% FBS was added under an environment of 37°C and 5% CO₂. Human umbilical cord blood CD34 positive hematopoietic stem cells were purchased from RIKEN BioResource Center.

### 2) Mice

NOD/Shi-scid, IL-2 RγKO Jic (NOD.Cg-Prkdc^{scid} Il2rg^{tm1Sug}/ShiJic) mice (hereinafter, referred to as NOG mice) were purchased from In-Vivo Science Inc. The mice were bred at a specific pathogen free (SPF) facility at Juntendo University.

### 3) Antibody and reagent

The following human antigen-specific antibodies were used for flow cytometry. BUV395 labeled anti-CD56 mAb (clone NCAM 16.2) and PE-labeled anti-CD26 mAb (clone MA261) were purchased from BD Biosciences. Brilliant Violet 421 labeled anti-CD4 mAb (clone RPA-T4), Brilliant Violet 510 labeled anti-CD14 mAb (clone M5E2), FITC-labeled anti-CD8 mAb (clone HIT8a), PerCP/Cy5.5 labeled anti-CD20 mAb (clone 2H7), APC-labeled anti-CD45 mAb (clone HI30), APC/Fire 750 labeled anti-CD3 mAb (clone SK7), Brilliant Violet 605 labeled anti-mouse CD45 mAb (clone 30-F11), and Human TruStain FcX and TruStain FcX (anti-mouse CD16/32) for blocking non-specific binding of the antibody were purchased from BioLegend. In addition, Brilliant Stain Buffer plus for suppressing non-specific binding between Brilliant Violets was purchased from BD Biosciences.

### (Consideration of ethics)

For studies using human umbilical cord blood CD34 positive hematopoietic stem cells, a study plan and the like for performing this study at the Juntendo University Graduate School of Medicine, a chair leader of which is the present inventor, have been submitted to the Ethics Review Committee, and approval (Juntendo University Medical Ethics Nos. 2017167 and 2020280) has been obtained. An animal experiment was performed based on so-called 3R, and an experimental plan was submitted to the Institutional Animal Care and Use Committee of the Juntendo University School of Medicine, where discussion was performed, and approval has been obtained (approval numbers: 2020270 and 2021056).

### (Example 1) Production of human immunized mice

In order for an ICI to exhibit an antitumor effect, presence of an immune system mainly including T cells is essential. Therefore, it is necessary to use a human immunized mouse in an experiment for examining a combined effect of a humanized anti-CD26 antibody and the ICI. In order to develop a new combined therapy utilizing an advantage of the humanized anti-CD26 antibody having few side effects, a human malignant mesothelioma cell line and a human lung cancer cell line cancer-bearing model using a human immunized mouse were produced.

### (1) Production of mice

A NOG mouse was irradiated with radiation at a low dose (100 cGy), and the next day, 1 × 10⁵ human umbilical cord blood CD34 positive hematopoietic stem cells were transferred into a tail vein of the mouse. Blood was collected from the mouse tail vein over time, and engraftment of human immune cells was confirmed.

In order to examine a combined effect of a humanized anti-CD26 antibody and a PD-1 antibody, a human immunized mouse in which human immune cells were engrafted was produced. In production of the mouse, it is necessary to irradiate a NOG mouse which is a severe immunodeficient mouse with radiation at a low dose and to transplant human hematopoietic stem cells, but it is considered that time from thawing to transplantation is very important even when hematopoietic stem cell transplantation is performed at a clinical site. A protocol published by RIKEN BioResource Center which is a supplier of the human umbilical cord blood CD34 positive hematopoietic stem cells and a protocol published by the Central Institute for Experimental Animals that developed NOG mice are different from each other in washing buffer, the number of times of washing (centrifugation), centrifugation time, and the like. Therefore, a protocol for stably engrafting human immune cells was examined.

### (1-1) Cell preparation

### a) Protocol of Central Institute for Experimental Animals(https://www.ciea.or.jp/laboratory_animal/pdf/NOG_hu HSC.pdf)

Frozen CD34 + cells (2.5 × 10⁵/tube) were thawed in a water bath at 37°C and transferred into a 50 mL conical tube. 1 mL of PBS containing 2% fetal bovine serum (2% FBS-PBS) was slowly added dropwise thereto while the tube containing the cells was shaken. 18 mL of 2% FBS-PBS was further added thereto, and then the mixture was centrifuged at 1,200 rpm for five minutes at room temperature. The collected cells were resuspended in 10 mL of 2% FBS-PBS and centrifuged again under the same conditions. The collected cells were resuspended in PBS.

### b) Protocol of RIKEN BioResource Center (Ishikawa, F., et al., Blood; 106: 1565-1573 (2005))

Frozen CD34 + cells (2.5 × 10⁵/tube) were thawed in a water bath at 37°C and transferred into a 50 mL conical tube. 15 mL of a washing solution (PBS) was added dropwise thereto at a rate of 1 drop/5 seconds. After mixing, 2 to 3 mL of PBS was further added thereto to make the total amount 20 mL. The mixture was centrifuged at 4°C and 300 G for 10 minutes. The collected cells were resuspended in PBS.

### c) Modified protocol by the present inventors

Frozen CD34 + cells (2.5 × 10⁵/tube) were thawed in a water bath at 37°C and transferred into a 50 mL conical tube. 15 mL of PBS containing 10% fetal bovine serum (100 FBS-PBS) was added dropwise thereto at a rate of 1 drop/5 seconds. After mixing, 3 to 4 mL of 10% FBS-PBS was further added thereto to make the total amount 20 mL. The mixture was centrifuged at 4°C and 1,300 rpm for five minutes. The collected cells were resuspended in PBS.

### (1-2) Introduction of cells into mice

0.2 mL (1 × 10⁵ cells) of cell suspension was injected into a tail vein of the NOG mouse irradiated with radiation at 100 cGy using a 29 G microinjection syringe.

### (2) Flow cytometry

In order to confirm engraftment of human immune cells in the mouse body, both of TruStain FcX for Human and TruStain FcX for Mouse were added to peripheral blood obtained by collecting blood from the tail vein of the mouse, and the mixture was stained with a fluorochromelabeled antibody. Thereafter, hemolysis and a fixation treatment were performed with BD FACS Lysing Solution (BD Biosciences), washing was performed, and then measurement was performed with BD LSRFortessa (BD Biosciences). The obtained data was analyzed with FlowJo (BD Biosciences).

### (3) Results

According to the protocol published by the Central Institute for Experimental Animals, human B cells were stably engrafted, but human T cells have not been generated even 20 weeks after transplantation (results are not illustrated). Meanwhile, in the protocol published by RIKEN BioResource Center, generation of human T cells was stably confirmed, but there was also a case where viability of CD34 + cells after thawing was low. Therefore, when the washing buffer and the centrifugation time were modified, generation of human T cells was confirmed without omission in all the examined mice in the protocol constructed by the present inventors. It was confirmed that until 10 weeks passed after transplantation of human hematopoietic stem cells, about 90% of human immune cells in blood of the mouse were B cells (CD20 positive), after the tenth week, a ratio of human CD4 T cells (CD3 positive CD4 positive) and CD8 T cells (CD3 positive CD8 positive) gradually increased, and about 20% of human blood cells were T cells in the 14th week, and about 30% of human blood cells were T cells in the 18th week (Fig. 1). In this model, a ratio of human NK cells (CD56 positive) was about 1 to 2%, and a ratio of human monocyte cells (CD14 positive) was about 1%.

### (Example 2) Combined use test using JMN cell-transplanted human immunized model mice

Using the mice produced in Example 1, a human malignant mesothelioma cell line JMN cancer-bearing model was produced, and a combined effect of an anti-CD26 antibody and an anti-PD-1 antibody was confirmed.

The malignant mesothelioma cell line JMN grows very slowly in vivo, and it takes five to six weeks from a time point when the malignant mesothelioma cell line JMN is subcutaneously transferred into a mouse to a time point when a tumor is formed. Therefore, the JMN cell line was subcutaneously transferred in the 13th week of hematopoietic stem cell transplantation when the number of human T cells increased in the mouse body.

13 weeks after transplantation of human hematopoietic stem cells, a cell suspension of JMN cells and Matrigel were mixed at a ratio of 1 : 1, and 1 × 10⁶ cells were subcutaneously transferred into a flank of each of the mice. From a time point when five weeks had elapsed after subcutaneous transfer of JMN and small tumor formation was confirmed, administration of each of control human IgG1 (Bio X Cell), a humanized anti-CD26 antibody (YS110) (Y's AC Co., Ltd.) alone, mouse anti-human PD-1 mAb (Bio X Cell; clone J116) alone, and a combination of the humanized anti-CD26 antibody and the PD-1 antibody three times a week at 200 µg/dose was started, and continued until dissection in the ninth week. A tumor size was measured twice a week, and a tumor volume was calculated by (width × height × height) × 1/2. Nine weeks after transfer of JMN, each of the mice was dissected, and a subcutaneous tumor thereof was collected and weighed. A part of the tumor was fixed with 10% formalin for pathological analysis, and the rest was subjected to an enzyme treatment with Liberase TL Research Grade (Roche) 0.25 mg/ml. A tissue was crushed in the presence of DNase I (Roche) to obtain cells in the tumor tissue. In analysis of tumor infiltrating lymphocytes, lymphocyte purification was performed using MagniSort Human CD3 Positive Selection Kit (invitrogen) and EasySep Magnet (STEMCELL). In addition, in order to compare properties with the tumor infiltrating lymphocytes, lymphocyte analysis of a spleen was also performed.

As a result of measuring the tumor size twice a week, regarding the tumor size nine weeks after transfer of JMN, suppression of tumor growth was observed in each of the humanized anti-CD26 antibody alone (YS alone) (average value 176.3 mm³) and the PD-1 antibody alone (PD1 alone) (average value 169.6 mm³) as compared with the control antibody administration group (average value 215.5 mm³), but the tumor size was smaller in the group of administering both antibodies (YS + PD1) (average value 123.6 mm³), and it was revealed that a synergistic effect was exhibited (Fig. 2).

Nine weeks after transfer of JMN, each of the mice was dissected, and a subcutaneous tumor thereof was collected. A part of the tumor was subjected to pathological analysis, the rest was used for purification of tumor infiltrating lymphocytes, and phenotypic analysis was performed. While no significant difference was observed between the control group and the YS alone group or between the control group and the PD1 alone group in both the tumor volume and the collected tumor weight at a time point of nine weeks when dissection was performed, a significant difference of p < 0.05 was observed between the control group and the YS + PD1 group (p = 0.0167, Fisher's multiple comparison test), and a combined effect of both antibodies was expected (Fig. 3).

### (Example 3) Combined use test using HCC4006 cell-transplanted human immunized model mice

Using the mice produced in Example 1, a human lung adenocarcinoma cell line HCC4006 cancer-bearing model was produced, and a combined effect of an anti-CD26 antibody and an anti-PD-1 antibody was confirmed.

14 weeks after transplantation of human hematopoietic stem cells, a cell suspension of HCC4006 cells and Matrigel were mixed at a ratio of 1 : 1, and 1 × 10⁶ cells were subcutaneously transferred into a flank of each of the mice. From a time point when three weeks had elapsed after subcutaneous transfer of HCC4006 and small tumor formation was confirmed, administration of each of control human IgG1 (Bio X Cell), a humanized anti-CD26 antibody (YS110) (Y's AC Co., Ltd) alone, mouse anti-human PD-1 mAb (Bio X Cell; clone J116) alone, and a combination of the humanized anti-CD26 antibody and the PD-1 antibody three times a week at 200 µg/dose was started, and continued until dissection in the 6.5th week. A tumor size was measured twice a week, and a tumor volume was calculated by (width × height × height) × 1/2. 6.5 weeks after transfer of HCC4006, each of the mice was dissected, and the subcutaneous tumor was collected and weighed. A part of the tumor was fixed with 10% formalin for pathological analysis, and the rest was subjected to an enzyme treatment with Liberase TL Research Grade (Roche) 0.25 mg/ml. A tissue was crushed in the presence of DNase I (Roche) to obtain cells in the tumor tissue. In analysis of tumor infiltrating lymphocytes, lymphocyte purification was performed using MagniSort Human CD3 Positive Selection Kit and EasySep Magnet. In addition, in order to compare properties with the tumor infiltrating lymphocytes, lymphocyte analysis of a spleen was also performed.

As a result of measuring the tumor size twice a week, regarding the tumor size six weeks after transfer of HCC4006, suppression of tumor growth was observed in each of the humanized anti-CD26 antibody alone (YS alone) (average value 272.3 mm³) and the PD-1 antibody alone (PD1 alone) (average value 236.0 mm³) as compared with the control antibody administration group (average value 445.2 mm³), but the tumor size was smaller in the group of administering both antibodies (YS + PD1) (average value 163.7 mm³), and it was revealed that a synergistic effect was exhibited (Fig. 4).

6.5 weeks after transfer of HCC4006, each of the mice was dissected, and a subcutaneous tumor thereof was collected. A part of the tumor was subjected to pathological analysis, the rest was used for purification of tumor infiltrating lymphocytes, and phenotypic analysis was performed. In both the tumor volume and the collected tumor weight at a time point of 6.5 weeks when dissection was performed, suppression of tumor growth was observed in each of the YS alone group and the PD1 alone group as compared with the control group, but the tumor size was smaller in the group of administering both antibodies (YS + PD1), and a combined effect of both antibodies was expected (Fig. 5).

## Claims

1. A pharmaceutical composition for treatment of cancer, comprising an immune checkpoint inhibitor and an anti-CD26 antibody or an antigen-binding fragment thereof as active ingredients.

2. A pharmaceutical composition for treatment of cancer, comprising an anti-CD26 antibody or an antigen-binding fragment thereof as an active ingredient for use in combination with an immune checkpoint inhibitor.

3. A pharmaceutical composition for treatment of cancer, comprising an immune checkpoint inhibitor as an active ingredient for use in combination with an anti-CD26 antibody or an antigen-binding fragment thereof.

4. The pharmaceutical composition according to any one of claims 1 to 3, wherein the immune checkpoint inhibitor and the anti-CD26 antibody or antigen-binding fragment thereof are administered simultaneously, continuously, or separately at a time interval.

5. The pharmaceutical composition according to any one of claims 1 to 4, wherein the cancer is a cancer expressing CD2 6.

6. The pharmaceutical composition according to any one of claims 1 to 5, wherein the immune checkpoint inhibitor and the anti-CD26 antibody or antigen-binding fragment thereof are used at a ratio of 1 : 1.

7. The pharmaceutical composition according to any one of claims 1 to 6, wherein the anti-CD26 antibody has a heavy chain consisting of the sequence set forth in SEQ ID NO: 17 and a light chain consisting of the sequence set forth in SEQ ID NO: 18.

8. The pharmaceutical composition according to any one of claims 1 to 7, wherein the immune checkpoint inhibitor is an anti-CTLA-4 antibody, an anti-PD-1 antibody, an anti-PD-L1 antibody, or an antigen-binding fragment thereof.

9. The pharmaceutical composition according to claim 8, wherein the immune checkpoint inhibitor is an anti-PD-1 antibody or an antigen-binding fragment thereof.
